Europäisches Patentamt

European Patent Office

Office européen des brevets

(19)

(11) Publication number: **0 195 672 B1**

## (12) EUROPEAN PATENT SPECIFICATION

(45) Date of publication of patent specification: **16.10.91**

(51) Int. Cl.⁵: **A61K 35/74**, C12N 15/00, A61K 37/54

(21) Application number: **86302044.2**

(22) Date of filing: **19.03.86**

The file contains technical information submitted after the application was filed and not included in this specification

(54) **Dental caries preventive preparations and method for preparing said preparations.**

(30) Priority: **20.03.85 JP 57443/85**

(43) Date of publication of application:
**24.09.86 Bulletin 86/39**

(45) Publication of the grant of the patent:
**16.10.91 Bulletin 91/42**

(84) Designated Contracting States:
**BE CH DE FR GB IT LI NL SE**

(56) References cited:
**EP-A- 0 100 254**
**FR-A- 2 502 958**

(73) Proprietor: **Matsushiro, Aizo**
**4-15-2, Aoyamadai**
**Suite 565(JP)**

(72) Inventor: **Matsushiro, Aizo**
**4-15-2, Aoyamadai**
**Suite 565(JP)**

(74) Representative: **Hayward, Denis Edward Peter**
**et al**
**Lloyd Wise, Tregear & Co. Norman House**
**105-109 Strand**
**London WC2R 0AE(GB)**

## Description

The present invention relates generally to recombinant methods and materials useful for securing the microbial expression of exogenous gene products. More particularly, the invention relates to microbial preparations for the prevention of dental caries and methods for their production.

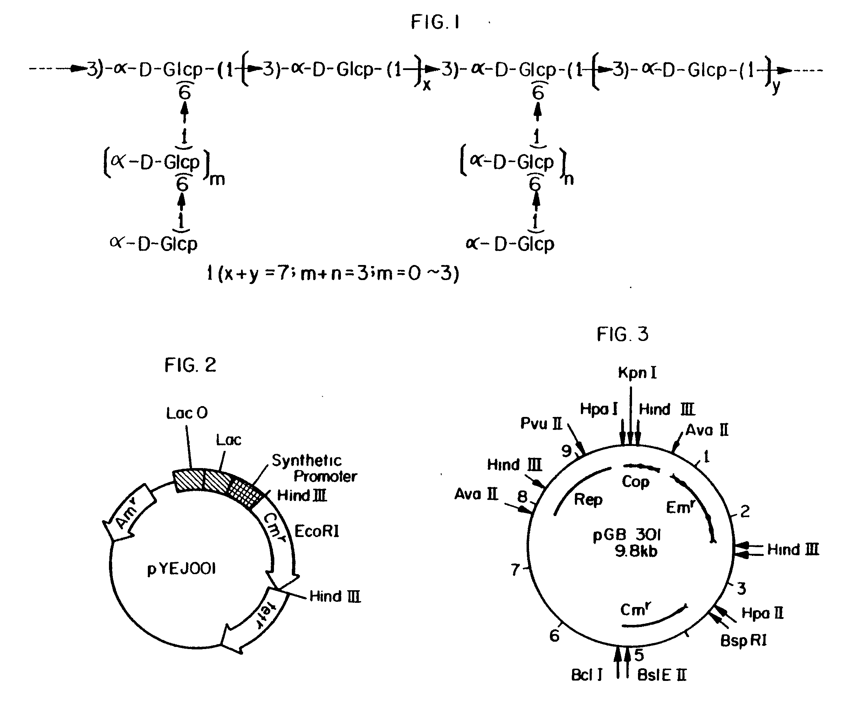

Streptococcus mutans, which causes dental caries, adheres to the surface of tooth enamel through the synthesis of extracellular polysaccharide polymers from sucrose. The most important of these polysaccharides are the glucans. One type of glucan contains predominantly α-1,6 bonds (see Figure 1) and is similar to the classical dextrans. The second type of glucan contains predominantly α-1,3 bonds and is less water soluble. S. mutans and other cariogenic microorganisms cause tooth decay by adhesion to the tooth surface and secretion of organic acids and other material which causes demineralization of the inorganic component of tooth structure and dissolution of the residual organic matrix.

In the past, dental plaque (which is a complex of cariogenic bacteria, insoluble glucans and other material) has been mechanically removed by brushing the teeth with a tooth brush and use of materials such as dental floss. It is significant, however, that bacteria sorb rapidly to the enamel surface within minutes after the teeth are vigorously cleaned and that macroscopically visible colonies will then appear within one or two days. In addition, it is extremely difficult to ensure thorough cleaning of the teeth as dental plaque will often remain intact in areas difficult to reach with a tooth brush or dental floss.

Recently, dentifrices have been developed which provide for the enzymatic decomposition of the α1,3 and α- 1,6 glucosidic bonds holding together insoluble glucan polymers. Such dentifrices typically comprise α-1,6 glucan 6-glucanohydrolase ( α-1,6 glucanase or dextranase) or α-1,3 glucan 3-glucanohydrolase (α-1,3 glucanase or mutanase). Yoshida, et al., United States Patent US-A-4,486,330 discloses cleaning compositions for artificial dentures comprising a beta-1,3 glucanase in a suitable buffer. Simonson, et al., United States Patent US-A-4,328,313 and Guggenheim, et al., United States Patent US-A-4,353,891 each disclose methods for production of plaque dispersing α-1,3 glucan 3-glucanohydrolase enzymes. Shimada, et al., United States Patent US-A-4,438,093 discloses an oral composition for the prevention and suppression of oral diseases comprising both α-1,3 glucanase and α-1,6 glucanase in a pharmaceutically acceptable carrier. The application of dentifrices containing enzymes for the disintegration of such insoluble glucan is not expected to have significant effects in decomposing dental plaque lasting beyond the period of brushing because most of the enzymatic components of the dentifrices tend to be lost during the rinsing which typically follows brushing.

To date, there have been no reports of the use of recombinant methods in the cloning and isolation of genes coding for α-1,3 or α-1,6 glucanase enzymes.

From the above description of the state of the art it is apparent that there exists a need in the art for improved methods and materials providing glucanase activity in the human oral cavity. Such methods and materials should preferably provide consistent long lasting glucanase activity and should preferably not require frequent application.

According to the present invention, there is provided a dental caries preventative preparation comprising a bacterium indigenous to the oral cavity which has been genetically transformed to express a glucanase enzyme.

The invention also provides a recombinant DNA molecule consisting of segments of DNA from different genomes which have been joined end-to-end outside of living cells and which have the capacity to transform a host and to be maintained therein, and the progeny thereof, comprising a DNA sequence selected from:

(a) a 3.0 kb EcoRI fragment of DNA present in Bacillus circulans BC-8 (FERM BP-733); and encoding α-1,3 glucan 3-glucanohydrolase,

(b) DNA sequences which hybridize to the foregoing DNA fragment and which code on expression for α-1,3 glucan 3-glucanohydrolase, and

(c) DNA sequences which code on expression for an α-1,3 glucan 3-glucanohydrolase enzyme of the type coded for on expression by any of the foregoing DNA sequences and fragments,

said DNA sequences and fragments being operatively linked to an expression control sequence in said recombinant DNA molecule.

Additionally provided are hosts transformed with such recombinant DNA molecules coding for the expression of 1-3 glucan 3-glucanohydrolase enzymes. Specific examples are Escherichia coli and Streptococcus hosts.

The present invention further provides a recombinant DNA molecule consisting of segments of DNA from different genomes which have been joined end-to-end outside of living cells and which have the capacity to transform a host and to be maintained therein, and the progeny thereof, comprising a DNA sequence selected from:

(a) a fragment of DNA present in Bacillus circulans BC-8 (FERM BP-733) or in the bacterium species denoted CB-8 deposited under accession number FERM BP-995 and encoding α-1,6 glucan 6-glucanohydrolase

(b) DNA sequences which hybridize to the foregoing DNA fragment and which code on expression for α-1,6 glucan 6-glucanohydrolase and

(c) DNA sequences which code on expression for an α-1,6 glucan 6-glucanohydrolase enzyme of the type coded for on expression by any of the foregoing DNA sequences and fragments,

said DNA sequences and fragments being operatively linked to an expression control sequence in said recombinant DNA molecule.

The aim being to provide methods and materials for the introduction and maintenance of long lasting glucanase activity in the oral cavity, the preferred technique comprises (1) cloning genes of a bacterium which produces α-1,3 glucanase, α-1,6 glucanase, or both of them, (2) transforming bacterial cells of a type indigenous to the oral cavity with such genes so as to allow for expression and secretion of the gene products, and (3) introducing the transformed bacteria into the oral cavity. The transformed organisms may be introduced to the oral cavity in a concentration such that sufficient levels of α-1,3 glucanase, α-1,6 glucanase or both of them are constantly expressed and secreted into the oral cavity so as to degrade the glucans of dental plaque and prevent the accumulation of such plaque and the adhesion of cariogenic organisms to the teeth. Use of both α-1,3 glucanase and α-1,6 glucanase in combination produces a synergistic effect in degrading the insoluble glucan materials making up dental plaque. Accordingly, genes coding for the expression and secretion of both types of glucanase may be introduced separately or together into the same or different transformed bacteria of the type indigenous to the oral cavity.

Procedures according to the invention will now be described by way of example and with reference to the accompanying drawings in which:-

Fig. 1 shows the structure of the insoluble glucan;

Fig. 2 is a gene map of the pYEJ 001 plasmid present in the cytoplasm of Escherichia coli;

Fig. 3 is a gene map of the pGB 301 plasmid present in the cytoplasm of Streptococcus sanguis.

Fig. 4 is a diagram showing the preparation of the shuttle vector pMN 1 which is obtained by cleaving the plasmid pGB 301 (9.8 kb) of S. sanguis and the plasmid pUC 9 (2.8 kb) of E. coli with Hae III and Sma I respectively, and ligating them with the T4 ligase;

Fig. 5 is a diagram showing the direction of transcription (reverse to the direction of Cmʳ gene which starts from the synthetic promoter) of the α-1,3 glucanase gene cloned in the pYEJ 001;

Fig. 6a is a diagram showing the preparation of pMN 2; and

Fig. 6b is a diagram showing the preparation of pMN 3.

The following examples illustrate isolation of bacteria expressing genes for glucanase activity, cloning and expression of genes coding for that activity, introduction of genes coding for activity into bacteria indigeneous to the oral cavity and the expression and secretion of glucanase gene products.

## EXAMPLE 1

This example relates to a procedure generally applicable for isolation of glucanase producing organisms from soil samples. More specifically, a sample of soil comprising weathered granite mixed with peat and having a pH of approximately 6 was obtained from the inventor's garden and was added to a sterilized and phosphate buffered (pH 7) minimal medium comprising 0.1% (by weight) $(NH_4)_2SO_4$, 0.0005% $MgSO_4 \cdot 7H_2O$ 0.0005% $FeC12 \bullet 6H_2O$ and 0.03% insoluble glucan obtained from cariogenic bacterium Streptococcus mutans strain OMZ 176 which was the sole carbon source. The soil samples were incubated in the medium for three consecutive days at 28°C. The method for preparing the insoluble glucan was in accordance with that of Ebisu (Osaka University Journal of Dentistry, Vol. 21, No. 1 1976).

A variety of bacteria cultured in the medium were subcultured twelve times and were concentrated. Agar plates were prepared comprising 1.2% (by weight) agar to which 0.2% insoluble glucan and the minimal medium described above had been added. The plates were then inoculated with the concentrated bacteria and were cultured to form colonies. After several days of incubation at 30°C, bacteria which expressed glucanase activity and degraded the insoluble glucan were identified by the formation of transparent halos around the colonies.

Four particular colonies identified as expressing glucanase activity were cultured and characterized. Bacteria forming the most conspicuous halo were identified as Bacillus circulans BC-8 and were deposited with Technical Research Institute of Microbial Industry, Agency of Industrial Science & Technology, Japan, under accession number FERM BP-733. The next most conspicuous halo was formed by a colony of bacteria denoted as CB8 and deposited under accession number FERM BP-995. Two colonies showing faint halos were identified as Pseudomonas sp.

## EXAMPLE 2

In this example, a determination was made of the activity of the glucanase enzyme produced by BC-8. In order to determine the activity of the glucanase enzyme, a suspension of the insoluble glucan of example 1 was pulverized by ultrasonic treatment and for use as a substrate. Bacillus circulans BC-8 (hereinafter "BC-8") was incubated in tripticase soy broth (Difco Laboratories, Detroit, Michigan) at 30°. Expression of the glucanase enzyme was induced only when the insoluble glucan was added to the broth. Enzyme-containing supernatant samples were obtained by centrifugation and 10-fold concentration of the culture medium. Alternatively, the supernatant was treated with 75% ammonium sulfate to induce precipitation of the enzyme. One unit of activity of enzyme was defined as that amount which produces 0.1 $\mu$M of glucose in 16 hours at 37°C from an insoluble glucan substrate.

Example 3

In this example, glucanase enzymes with $\alpha$-1,3 and $\alpha$-1,6 activities were produced by the culture of bacterium BC-8. Cultures of bacterium BC-8 were incubated in 500 ml of tripticase soy broth containing 0.3% insoluble glucan for three days at 30°C. The culture supernatant obtained by centrifugation was treated with 75% ammonium sulfate solution to precipitate the enzyme. The resulting precipitate was dissolved in 50 mM phosphate buffer solution (pH 7.0), and this solution was then added to a DE-52 cellulose column. Fractions were collected while the column was eluted with 50mM phosphate buffer solution (pH 7.0) with NaCl increasing in concentration from 0 to 0.5 molar.

The fraction of this eluate showing peak activity was then subjected to the Sephadex® G-150 column chromatography. SDS-gel electrophoresis revealed the presence of two bands, one 68 kilodaltons (kd) and the other 54 kd in molecular weight. Of these bands, the protein of the 68 kd band was identified as an $\alpha$-1,3 glucanase while the protein of the 54 kd band was identified as having $\alpha$-1,6 glucanase activity.

The 68 kd. protein was purified in the manner described above and was applied to glucan which had had its $\alpha$-1,6 linkages destroyed by treatment with periodic acid or commercially available dextranase thereby having only $\alpha$-1,3 linkages. The 68 kd protein was found to degrade the $\alpha$-1,3 bonds to produce reducing sugars. When a commercially available dextran with $\alpha$-1,6 linkages was subjected to the 68 kd enzyme, almost no reducing sugars were produced thus indicating that the enzyme was an $\alpha$-1,3 glucanase. Hexokinase added during the enzymatic treatment of the glucan detected the presence of glucose monosaccharide thus indicating that the glucanase was an endo type of glucanase formally classified as $\alpha$-1,3 glucan 3-glucanohydrolase. Similar tests conducted on the 54 kd enzyme found that that enzyme had glucanase activity specific for the $\alpha$-1,6 linkage and was an $\alpha$-1,6 glucan 6-glucanohydrolase.

While it is known that the linkages of insoluble glucan are predominantly of the $\alpha$-1,3 variety and that $\alpha$-1,3 glucanase plays the principal role in enzymatic degradation of insoluble glucan it has been found that the combination of the $\alpha$-1,3 glucanase and the $\alpha$-1,6 glucanase has synergistic effects on the degradation of insoluble glucans. Accordingly, to remove insoluble glucan, it is desirable to clone both genes for $\alpha$-1,3 glucanase and for $\alpha$-1,6 glucanase and link them into one plasmid.

EXAMPLE 4

In this example, the gene coding for the expression of the BC-8 $\alpha$-1,3 glucanase was cloned into an E. coli expression vector and transformed into E. coli. Bacterium BC-8 was cultured with tripticase soy broth and the DNA was extracted in accordance with the method of Marmur, J. Mol. Bio., Vol. 3, pp. 208, 1961. This material was centrifuged by the CsCl-EtBr equilibrium density gradient centrifugation method which revealed no plasmid DNA. A single band of chromosome DNA was then isolated and purified. The DNA thus purified was then dialyzed and cleaved by EcoRI endonuclease.

At the same time, E. coli HB 101 having the commercially available expression vector pYEJ 001 plasmid (Pharmacia P-L Biochemicals, Uppsala, Sweden) (see Fig. 2) was cultured with 300 ml of L-broth [comprising 10g peptone, 5g yeast extract, 1 g glucose, 5 g NaCl and 1000 ml of $H_2O$ (adjusted to pH 7.2)]. The pYEJ 001 plasmid DNA was then extracted and isolated and itself cleaved by EcoRI.

One $\mu$g of the bacterium BC-8 EcoRI DNA fragments and one $\mu$g of the pYEJ 001 plasmid DNA thus obtained were then combined in the presence of one unit of T4 ligase and incubated for 12 hours at 4°C. The recombinant plasmid DNA thus generated by the combination of the two DNA fragments was then dialyzed in 10 mM Tris-HCl solution (pH 7.5, 1 mM EDTA). The recombinant plasmid DNA was then transformed into E. coli K12 strain HB 101.

EXAMPLE 5

In this example, transformed E. coli K12 strain HB 101 bacteria were screened for the presence of the $\alpha$-1,3 glucanase gene.

As shown in Fig. 2, if a DNA fragment is inserted into the EcoRI site of the chloramphenicol resistance gene (Cm$^r$) of the pYEJ 001 plasmid, the bacterium will become sensitive to chloramphenicol. In addition, because the synthetic promoter is present upstream of this site, any gene in the DNA inserted into that site which is aligned in the proper (3' to 5') direction and in the proper reading frame can be strongly expressed.

Cultures of E. coli which were transformed with the recombinant pYEJ 001 plasmid were arranged to make colonies on agar plates containing ampicillin, and each colony was transferred by a sterilized stick to a L-agar plate (L-broth containing 1.5% agar) containing chloramphenicol (Cm). Cultures of E. coli showing Cm sensitivity were those into which some DNA fragment was inserted at the EcoRI site.

From among the colonies showing Cm sensitivity, it was then necessary to identify those into which the α-1,3 glucanase gene had been successfully inserted. Because E. coli is a gram-negative bacterium (and unlike bacterium BC-8, is incapable of secreting the glucanase enzyme) no transformants were found to secrete glucanase out of several thousands of colonies transferred to plates containing insoluble glucan.

As the host bacterium HB 101 requires amino acids such as threonine, leucine and proline, the Cm-sensitive (Cm$^s$) E. coli colonies were cultivated on a synthetic minimal medium comprising a small amount of casamino acids and 0.2% ultrasonically pulverized insoluble glucan as the only carbon source. While no halos were formed and the transformants were deemed incapable of secreting the α-1,3 glucanase, the fact that some of the colonies were observed to grow indicated that they expressed the product of the α-1,3 glucanase gene and had acquired the capability to degrade and exploit the insoluble glucan as a carbon source.

Those transformants observed to grow on the minimal medium were then cultured on a slightly larger scale (1 liter), and their plasmid DNA was extracted and cleaved by EcoRI endonuclease. The digested DNA was then examined by gel electrophoresis and found to contain a 3.0 kb DNA fragment. This fragment size was considered sufficient to code for a gene product the size of a 68 kd molecular weight protein calculated for the α-1,3 glucanase material identified in example 3.

To demonstrate that the α-1,3 glucanase gene is present in this 3.0 kb DNA fragment, various bacterial cultures were incubated in 30 ml of minimal media containing 0.2% insoluble glucan. The cultures included; (1) E. coli HB 101; (2) Bacterium HB 101 transformed with plasmid pYEJ 001; and (3) Bacterium HB 101 transformed with plasmid pYEJ 001 including the 3.0 kb DNA fragment insert. The supernatant obtained from centrifugation of lysed cells from the three types of cultures was assayed for glucanase activity according to the methods of example 2 with the result that only the bacteria transformed with plasmid pYEJ 001 including the 3.0 kb fragment exhibited glucanase activity thus demonstrating that the α-1,3 glucanase gene of the bacterium BC-8 had been successfully cloned into the pYEJ 001 plasmid of E. coli. Further, only HB 101 cells transformed with pYEJ 001 including the 3.0 kb insert were able to survive in casamino acids and insoluble glucan as described above.

EXAMPLE 6

In this example, Streptococcus sanguis Challis strain (NCTC7868) a bacterium normally present in the flora of the oral cavity was successfully transformed with the gene coding for the expression of α-1,3 glucanase. Among various bacteria indigenous to the oral cavity, S. sanguis and Streptococcus salivarius are the most innocuous bacteria, and in particular, Streptococcus sanguis Challis strain (NCTC7868) is a material the genetics and transformation of which are relatively well understood. Plasmid pGB 301 (see Fig. 3, Behnke, et al.; M.G.G. Molecular and Genetics, 184, 115-120 (1981); Behnke, et al., Microbiology, American Society for Microbiology, 239-242 (1982) was used as a transformation vector.

As shown in Fig. 3, the pGB 301 plasmid DNA which is present in the cytoplasm of S. sanguis Challis strain has two drug-resistant markers, Em$^r$ (erythromycin resistance) and Cm$^r$ (chloramphenicol resistance) with a unique Bst E II restriction site within the Cm$^r$ gene. The pGB 301 plasmid DNA was cleaved by the restriction enzyme Bst E II, and was blunt ended with DNA polymerase I.

At the same time, the pYEJ 001 plasmid containing the 3.0 kb fragment coding for the expression of α-1,3 glucanase gene was treated with EcoRI endonuclease and the 3.0 kb fragment was blunt ended with DNA polymerase I. The pGB 301 plasmid DNA fragment and the α-1,3 glucanase gene DNA fragment were then mixed at a ratio of 1 μg to 1 μg, and their blunt ends were ligated with 100 units of T4 ligase to reform a plasmid. In the recombinant plasmid thus obtained, the Cm$^r$ gene is cleaved, but the inserted α-1,3 glucanase gene is intact and should achieve phenotypic expression.

Accordingly, the S. sanguis Challis strain was transformed by insertion of the modified pGB 301 plasmid containing the 3.0 kb fragment bearing the α-1,3 glucanase gene. The transformation was carried out in accordance with the procedures of Le Blanc & Hassell [J. of Bacteriol., 128(1), 347-355 (1976)] and Macrina, et al. [Infec. & Imm., 28(3), 692-699 (1980)]. Colonies of the S. sanguis Challis strain which received the pGB 301 plasmid containing the α-1,3 glucanase gene were cultured on the Brain Heart Infusion (B.H.I.) agar plates (Difco Laboratories) containing erythromycin (50 μg/ml). Each of these colonies was transferred to a B.H.I. agar plate containing chloramphenicol (10 μg/ml) to examine for the presence of Cm$^s$ (chloramphenicol-sensitive) colonies. Most of the S. sanguis Challis colonies showing chloramphenicol sensitivity were expected to have the α-1,3 glucanase gene inserted into the pGB 301 plasmid.

Even for those S. sanguis Challis strains showing chloramphenicol sensitivity, phenotypic expression of the inserted α-1,3 glucanase gene was observed in only about one third of the transformed colonies. This is because the gene fragment can be inserted in two different orientations. Expression of the gene product was tested for by transfer of the Cm$^s$ bacterium colonies by a sterilized stick to B.H.I. agar plates containing insoluble glucan. Because S. sanguis is a gram-positive bacterium it would be expected to secrete any α-1,3 glucanase that it would produce. Expression and secretion of the glucanase was detected by the presence of halos on the glucan plate. The α-1,3 glucanase gene was thus successfully introduced into the cells of S. sanguis, a bacterium which is normally present in the oral cavity, and the phenotypic expression of the gene was achieved in that host.

EXAMPLE 7

In this example, the gene coding for the expression of the α-1,3 glucanase gene was introduced into S. sanguis using plasmid pMN 1. While plasmid pGB 301 (Figure 3) has two stable drug-resistant markers, Em$^r$ (erythromycin resistance) and CM$^r$ (chloramphenicol resistance), and is a relatively small plasmid, there are several limitations on its efficient use. First, the copy number of pGB 301 in a S. sanguis cell is only about ten. Second, as a result of the low copy number, it is rather inefficient to culture small quantities of S. sanguis having plasmid pGB 301 and to make simple checks of the plasmid size or to culture large quantities of transformed S. sanguis and obtain the plasmid DNA.

As a result of these limitations, it was decided to combine pGB 301 and E. coli plasmid pUC 9 in order to make up an improved transformation system. Plasmid pUC 9 (Pharmacia, Uppsala, Sweden) is one of the smallest plasmids of E. coli, has a high copy number and has several polycloning sites. The pGB 301 plasmid was opened by cleavage at a unique SmaI restriction site and pUC 9 was opened by cleavage at a unique SmaI site to produce, in both cases, linear molecules having blunt ends on both ends. The DNA fragments were then mixed and ligated by application of T4 DNA ligase to produce recombinant plasmids.

The resulting plasmids were transformed into E. coli strain JM 103 (PI)$^-$. E. coli bacteria successfully transformed and containing the lac Z marker of pUC 9 were detected as white colonies among blue colonies on an appropriate indicator plate by addition of the 5-bromo-3-choroindoyl-B-D-galactoside indicator to selective media according to the methods of Messing, et al., Proc. Natl. Acad. Sci. U.S.A., 74, 3642 (1977) and Messing, et al., Gene, 19, p. 276 (1982).

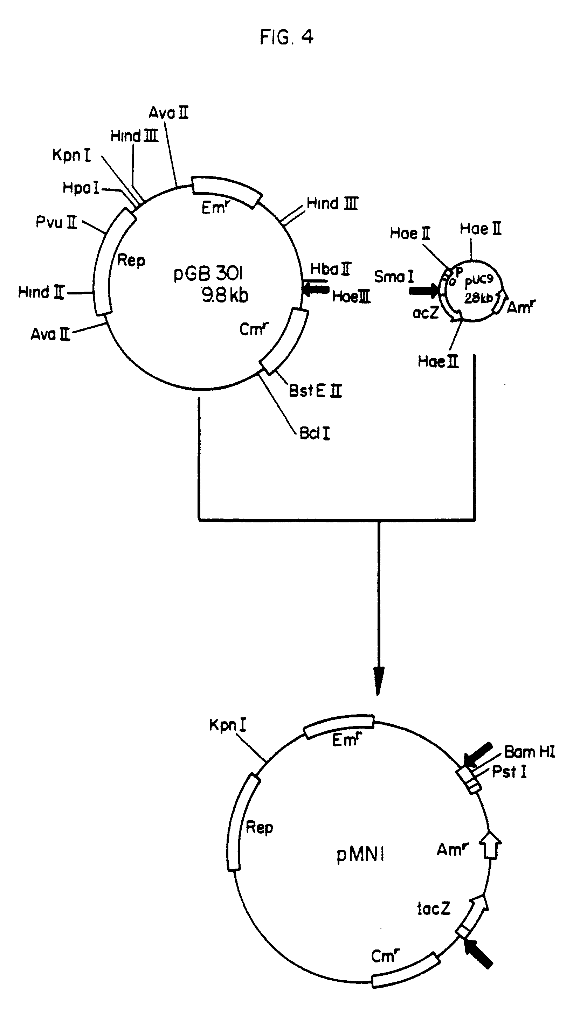

Plasmids were extracted from those colonies identified as containing the lac Z marker and one identified as having a size of 12 kb [equivalent to the sum of pGB 301 (9.8 kb) and pUC 9 (2.8 kb)] and the arrangement shown in Fig. 4 was designated as pMN 1. It can then be used as a cloning vector for the α-1,3 glucanase gene as described in example 8.

The plasmid was then used as a shuttle vector to transform S. sanguis as described above, conferring resistance to ampicillin as well as to chloramphenicol and erythromycin in transformed S. sanguis.

In example 5 it was noted that the α-1,3 glucanase gene was inserted downstream of the synthetic promoter of the pYEJ 001 plasmid. Surprisingly, the expression activity was not as strong as was expected. It was then suspected that the α-1,3 glucanase gene was inserted counter to the regular direction relative to the synthetic promoter and expression was in fact regulated by an endogenous Bacillus promoter present on the EcoRI fragment. Accordingly, an experiment was conducted to determine the direction of insertion of the α-1,3 glucanase gene.

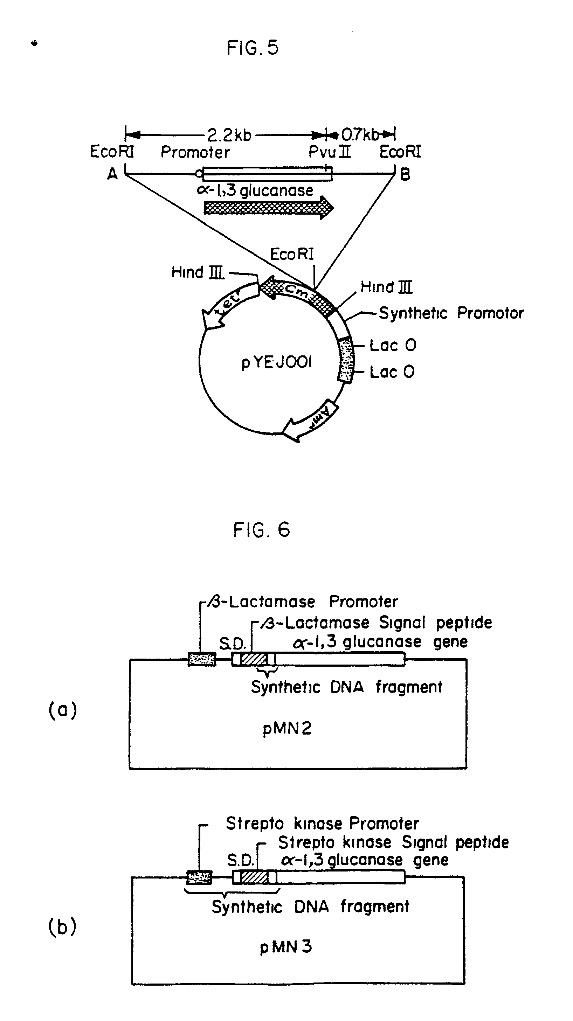

The α-1,3 glucanase gene inserted into plasmid pYE 001 (see Figure 5) may be transcribed in vitro by the RNA polymerase of E. coli. More than twenty cultures which contained a 3.0 kb fragment cloned to the EcoRI site of plasmid pYE 001 were evaluated and on the basis of the cleaving distance from the PvuII endonuclease site, it was concluded that in all cases the α-glucanase gene was present in the reverse orientation and mRNA transcription progresses from A to B.

Statistically, in approximately one half of the cases, the α-1,3 glucanase gene should have been inserted in the regular direction relative to the synthetic promoter, that is proceeding B to A. In this configuration, a powerful phenotypic expression would have been expected and large quantities of α-1,3 glucanase would be expected to be produced. Judging from the fact that no such strains were isolated it appears that the signal peptide of the α-1,3 glucanase gene product of bacterium BC-8 was not cut away by the signal peptidase of E.

coli. Further it appears that if the production of $\alpha$-1,3 glucanase under the control of the powerful synthetic promoter is excessive, the host bacteria accumulating $\alpha$-1,3 glucanase in the cells will die.

In those cases where the $\alpha$-1,3 glucanase gene was inserted in the reverse direction, that is A to B , transcription by the regular promoter of the $\alpha$-1,3 glucanase gene of the bacterium BC-8 will take place. The transcription seems to be reduced to a very low level by the powerful competitive effect of transcription by the synthetic promoter. This appears to be confirmed by experiments in which when the $\alpha$-1,3 glucanase gene was inserted, under non-inductive conditions, downstream of promoters of strong inductive systems, such as the tryptophan promoter (Ptrp) or lactose promoter (Plac) of E. coli. In these cases products of genes inserted in both regular and reverse directions were obtained. The genes inserted in the regular direction exhibited considerably stronger production of $\alpha$-1,3 glucanase.

This observation suggests that in order to efficiently produce the glucanase, the secretion signal peptide portion of the $\alpha$-1,3 glucanase gene product should be modified such that the signal peptide may be readily cleaved off of the glucanase enzyme and the $\alpha$-1,3 glucanase gene should be inserted in the "regular" direction downstream of a strong promoter.

EXAMPLE 8

This example provides procedures for construction of plasmids coding for fusion proteins comprising the secretion signal peptide sequence of $\beta$-lactamase and the "mature", signal-free polypeptide product of the $\alpha$-1,3 glucanase gene. It has been found that $\beta$-lactamase, a product of the ampicillin resistance (Am$^r$) gene, is expressed effectively in E. coli as well as in S. sanguis.

Plasmid pMN 1 is manipulated to include the glucanase gene in a similar manner to that used with pGB 301. That is, pMN 1 is cut with BstEI endonuclease and blunt ended. At the same time, plasmid pYEJ 001 is cut with EcoRI endonuclease to remove the 3.0 kb glucanase gene fragment which is blunt ended. This fragment is then ligated to the linear pMN 1 fragment using T4 ligase.

A gene fragment comprising the promoter and secretion signal peptide of $\beta$-lactamase is cleaved from plasmid pGH 54 modified by Ohgai, et al., Annual Meeting of Japanese Molecular Biology Assn., Tokyo, Japan, Dec. 4, 1985, and is inserted into a NurI site within the DNA encoding the $\alpha$-1,3 glucanase gene on pMN 1 into which the gene has been inserted.

The NurI site has been found to exist in the region of the glucanase gene coding for the amino terminal of the enzyme polypeptide. In order to complete this construction, it is necessary to employ a synthetic linker serving to connect the $\beta$-lactamase signal peptide encoding DNA on the pGH 54 fragment to the remainder of the glucanase gene (3' to the NurI site) and build back the DNA (5' to the NurI site) encoding the amino terminal of the enzyme. The resulting plasmid (designated pMN 2) is schematically represented in Figure 6a.

EXAMPLE 9

In this example plasmids are constructed coding for the expression of a fusion protein comprising the secretion signal peptide for streptokinase and the "mature", signal-free polypeptide product of the $\alpha$-1,3 glucanase gene. A gene fragment comprising the promoter and secretion signal peptide for streptokinase was cloned from Streptococcus equicimilis and its base sequence was determined (Malke & Ferretti, Proc. Natl. Acad. Sci. U.S.A., 81, 3557-3561 (1984); Malke, et al., Gene, 34, 357-362 (1985). This gene sequence is known to code for the secretion of streptokinase by cells of S. equicimilis as well as by E.coli. Plasmid pMN 3 (see Fig. 6b) is obtained by synthesizing a DNA sequence corresponding to the promoter and signal peptide of this enzyme, as well as the glucanase gene fragment, and ligating the synthesized DNA sequence into the NurI site in a pMN 1 plasmid manipulated to include the gene coding for the $\alpha$-1,3 glucanase protein.

Cultures of E. coli strain JM 103 and S. sanguis may be transformed according to the methods disclosed above with plasmids pMN 2 and pMN 3 and are expected to express both erythromycin and ampicillin resistance. The transformed cells are expected to have a considerable quantity of $\alpha$-1,3 glucanase accumulated in their periplasm and the transformed S. sanguis cells are expected to secrete the enzyme.

In this way, phenotypic expression of the $\alpha$-1,3 glucanase gene is expected to be successfully demonstrated in S. sanguis which is a bacterium indigeneous to the oral cavity. As previously disclosed, insertion of the glucanase gene in the regular direction relative to the promoter, and replacement of the signal peptide with one that functions in the cells of S. sanguis are important to the expression and secretion of large quantities of $\alpha$-1,3 glucanase.

Methods have thus far been disclosed for achieving phenotypic expression of the $\alpha$-1,3 glucanase gene by cloning the gene and introducing it into bacteria indigeneous to the oral cavity. As previously disclosed in example 1 above, the bacterium B. circulans BC-8 (FERM BP-733) is a bacterium which produces α-1,3 glucanase as well as α-1,6 glucanase. It therefore is possible to clone the α-1,6 glucanase gene by methods similar to those disclosed in the examples above in which dextran would be employed as a substrate. Alternatively, isolation of the α-1,6 glucanase gene from CB-8 (FERM BP-995) is projected. The bacterium CB-8 was isolated from a soil sample from the same area as that from which BC-8 was isolated. Bacteria in this sample were cultured using the same minimal medium as in Example 1, except that commercial dextran (Wako Junyaku K.K., Japan) having a molecular weight of 100,000-200,000 replaced insoluble glucan as the carbon source. Subcultured bacteria were then incubated on 0.5% blue dextran and 0.5% dextran. Four strains formed conspicuous halos. Three were identified as Actinomyces sp. The fourth, sp. CB-8, deposited under accession number FERM BP-995, will be employed as a source of the α-1,6-glucanase gene for cloning into S. sanguis so as to achieve phenotypic expression of the α-1,6 glucanase.

Novel DNA sequences provided by the present invention are useful not only in securing glucanase enzyme production in heterologous host cells, but are useful as hybridization probes for isolation of glucanase enzyme encoding genes of various microbial species by well known means. In addition to the cloning of α-1,3 glucanase gene isolated from B. circulans BC-8 it is also possible to clone the gene coding for α-1,3 glucanase isolated from Pseudomonas SK-01 (FERM P-No. 4273) and Pseudomonas (NRRL B-12324), as well as the α-1,6 glucanase gene from bacteria which produce α-1,6-glucanase such as Corynebacterium AK-01 (FERM P-No. 2505), Flavobacterium BK-01-06 (FERM P-No. 1194 or FERM P-No. 1285~1288), Paecilomyces TCI-No. 9001 (FERM P-No. 6602), and Penicillium pheniculosum IAM-7013 (FERM P-No. 1290) by gene manipulation techniques similar to those disclosed above. Glucanase genes so isolated may then be introduced into cells of S. sanguis in order to promote phenotypic expression of the glucanase enzymes.

Furthermore, as disclosed in example 5 above, the combination of α-1,3 glucanase and α-1,6 glucanase has synergistic properties and is very effective in removing insoluble glucan. It would be possible to remove insoluble glucan most effectively by preparing a plasmid in which the α-1,3 glucanase gene and the α-1,6 glucanase gene are present in series downstream of a powerful promoter (e.g. β-lactamase or streptokinase promoter) on a plasmid and introducing it into S. sanguis so as to produce the two enzymes simultaneously. Genes encoding one or both enzymes may also be inserted into the genome of the desired host.

Bacteria other than S. sanguis which are indigenous to the oral cavity and might be useful in the present invention include other species of Streptococcus such as Streptococcus salivarius. It is possible to introduce the gene to these bacteria by methods similar to those disclosed for transforming S. sanguis.

As previously disclosed, the dental caries preventative preparations of the present invention comprise a bacterium indigenous to the oral cavity, which bacterium has been transformed by introduction of the α-1,3 glucanase gene, the α-1,6 glucanase gene, or both. Said genes produce enzymes which disintegrate the insoluble glucan produced by the cariogenic bacteria which cause dental caries. Accordingly, the dental caries prevention preparation of the present invention may be adhered to the teeth by appropriate means such that expression and secretion of glucanase enzymes will act to essentially continuously disintegrate the insoluble glucan produced by the cariogenic bacterium in the oral cavity. Moreover, the glucanase enzymes will be produced constantly, and as a result, dental caries will be prevented reliably. It has been reported, for example, that S. sanguis incubated on teeth were fixed for more than two years and six months at the incubation site. See, Svanberg, et al., Archs. Oral. Biol., 31(1), 1-4 (1986).

## Claims

1. A dental caries preventative preparation comprising a bacterium indigenous to the oral cavity which has been genetically transformed to express a glucanase enzyme.

2. The preparation of Claim 1, wherein the bacterium has been genetically transformed to secrete the glucanase enzyme.

3. The preparation of Claim 1 or Claim 2, wherein the enzyme is α-1,3 glucan 3-glucanohydrolase.

4. The preparation of Claim 1 or Claim 2, wherein the enzyme is α-1,6 glucan 6-glucanohydrolase.

5. The preparation of any preceding claim, wherein the bacterium is a gram-positive bacterium.

6. The preparation of Claim 5 wherein the bacterium is a member of the genus Streptococcus.

7. The preparation of Claim 6 wherein the bacterium is a Streptococcus sanguis.

8. The preparation of Claim 6 wherein the bacterium is a Streptococcus salivarius.

9. A recombinant DNA molecule consisting of segments of DNA from different genomes which have been joined end-to-end outside of living cells and which have the capacity to transform a host and to be maintained therein, and the progeny thereof, comprising a DNA sequence selected from:
   (a) a 3.0 kb EcoRI fragment of DNA present in Bacillus circulans BC-8 (FERM BP-733); and encoding $\alpha$-1,3 glucan 3-glucanohydrolase,
   (b) DNA sequences which hybridize to the foregoing DNA fragment and which code on expression for $\alpha$-1,3 glucan 3-glucanohydrolase, and
   (c) DNA sequences which code on expression for an $\alpha$-1,3 glucan 3-glucanohydrolase enzyme of the type coded for on expression by any of the foregoing DNA sequences and fragments,
   said DNA sequences and fragments being operatively linked to an expression control sequence in said recombinant DNA molecule.

10. The recombinant DNA molecule according to Claim 9, wherein the molecule comprises a cloning vehicle having at least one restriction endonuclease recognition site, said DNA sequence being inserted at one of said recognition sites or between two of such sites.

11. The recombinant DNA molecule according to Claim 10, wherein the expression control sequence is also inserted into the cloning vehicle.

12. A unicellular host transformed with at least one recombinant DNA molecule according to any of Claims 9 to 11.

13. The transformed host according to Claim 12, wherein the host is E. coli.

14. The transformed host according to Claim 12, wherein the host is a gram-positive bacterium.

15. The transformed host according to Claim 14, wherein the bacterium is a member of the genus Streptococcus.

16. The transformed host according to Claim 15, wherein the bacterium is a Streptococcus sanguis.

17. The transformed host according to Claim 15, wherein the bacterium is a Streptococcus salivarius.

18. A recombinant DNA molecule consisting of segments of DNA from different genomes which have been joined end-to-end outside of living cells and which have the capacity to transform a host and to be maintained therein, and the progeny thereof, comprising a DNA sequence selected from:
   (a) a fragment of DNA present in Bacillus circulans BC-8 (FERM BP-733) or in the bacterium species denoted CB-8 deposited under accession number FERM BP-995 and encoding $\alpha$-1,6 glucan 6-glucanohydrolase
   (b) DNA sequences which hybridize to the foregoing DNA fragment and which code on expression for $\alpha$-1,6 glucan 6-glucanohydrolase and
   (c) DNA sequences which code on expression for an $\alpha$-1,6 glucan 6-glucanohydrolase enzyme of the type coded for on expression by any of the foregoing DNA sequences and fragments,
   said DNA sequences and fragments being operatively linked to an expression control sequence in said recombinant DNA molecule.

## Revendications

1. Préparation prévenant les caries dentaires comprenant une bactérie résidant dans la cavité buccale, qui a été transformée par voie génétique pour exprimer une enzyme glucanase.

2. Préparation suivant la revendication 1, dans laquelle la bactérie a été transformée pour sécréter l'enzyme glucanase.

3. Préparation suivant la revendication 1 ou 2, dans laquelle l'enzyme est $\alpha$-1,3-glucane 3-glucanohydrolase.

4. Préparation suivant la revendication 1 ou 2, dans laquelle l'enzyme est $\alpha$-1,6-glucane 6-glucanohydrolase.

5. Préparation suivant l'une quelconque des revendications précédentes, dans laquelle la bactérie est une bactérie gramm-positive.

6. Préparation suivant la revendication 5, dans laquelle la bactérie est un élément du gène Streptococcus.

7. Préparation suivant la revendication 6, dans laquelle la bactérie est un Streptococcus sanguis.

8. Préparation suivant la revendication 6, dans laquelle la bactérie est un Streptococcus salivarius.

9. Recombinant de molécule d'ADN consistant en segments d'ADN de différents génomes qui ont été réunis bout à bout à l'extérieur de cellules vivantes et qui ont la capacité de transformer un hôte et d'y être maintenue, et son progène, comprenant une séquence d'ADN choisie à partir de:

a) un fragment 3,0 kb EcoRI d'ADN présent dans Bacillus circulans BC-8 (FERM BP-733) ; et encodage α-1,3 glucane 3-glucanohydrolase,
b) des séquences d'ADN qui deviennent hybrides vers le fragment d'ADN précédent et qui codent sur expression pour α-1,3-glucane 3-glucanohydrolase, et
c) des séquences d'ADN qui codent sur expression pour une enzyme α-1,3-glucane 3-glucanohydrolase du type codé pour une expression par une des séquences d'ADN précédentes et des fragments, ces séquences d'ADN et les fragments étant liés en fonctionnement à une séquence de commande de l'expression dans cette molécule d'ADN recombinant.

10. Molécule d'ADN recombinant selon la revendication 9, dans laquelle la molécule comprend un vecteur de clonage ayant au moins un site de reconnaissance d'endonucléase de restriction, cette séquence d'ADN étant insérée sur l'un des sites de reconnaissance ou entre deux de ces sites.

11. Molécule d'ADN recombinant selon la revendication 10, dans laquelle la séquence de commande de l'expression est également insérée dans le vecteur de clonage.

12. Hôte unicellulaire transformé par au moins une molécule d'ADN recombinant selon l'une des revendications 9 à 11.

13. Hôte transformé selon la revendication 12, l'hôte étant E.coli.

14. Hôte transformé selon la revendication 12, l'hôte étant une bactérie gramm-positive.

15. Hôte transformé selon la revendication 14, la bactérie étant un élément du gène Streptococcus.

16. Hôte transformé selon la revendication 15, la bactérie étant un Streptococcus sanguis.

17. Hôte transformé selon la revendication 15, la bactérie étant un Streptococcus salivarius.

18. Molécule d'ADN recombinant se composant de segments d'ADN de différents génomes, qui ont été réunis bout à bout à l'extérieur de cellules vivantes et qui ont la capacité de transformer un hôte et de s'y maintenir, et son progène, comprenant une séquence d'ADN choisie parmi:

a) un fragment d'ADN présent dans le Bacillus circulans BC-8 (FERM BP-733) ou dans l'espèce de bactérie appelée CB-8 déposée sous le numéro d'accès FERM BP-995 et encodant l'α-1,6 glucane 6-glucanohydrolase,
b) des séquences d'ADN qui hybrident le fragment d'ADN précédent et qui codent sur expression pour α-1,6 glucane 6-glucanohydrolase, et
c) des séquences d'ADN qui codent sur expression pour une enzyme α-1,3-glucane 3-glucanohydrolase du type codé pour une expression par une des séquences d'ADN précédentes et des fragments, ces séquences et fragments d'ADN étant liés en fonctionnement à une séquence de commande d'expression dans cette molécule d'ADN recombinant.

**Patentansprüche**

1. Schutzpräparat gegen Zahnkaries, das ein im Mundraum natürlich vorkommendes Bakterium aufweist, das genetisch transformiert wurde, um ein Glukanase-Enzym zu exprimieren.

2. Präparat nach Anspruch 1, dadurch **gekennzeichnet,** daß das Bakterium genetisch transformiert wurde, um das Glukanase-Enzym zu sezernieren.

3. Präparat nach einem der vorhergehenden Ansprüche 1 oder 2, dadurch **gekennzeichnet,** daß das Enzym α-1,3 Glukan-3-glukanhydrolase ist.

4. Präparat nach einem der vorhergehenden Ansprüche 1 oder 2, dadurch **gekennzeichnet,** daß das Enzym α-1,6 Glukan-6-glukanhydrolase ist.

5. Präparat nach einem der vorhergehenden Ansprüche 1 bis 4, dadurch **gekennzeichnet,** daß das Bakterium ein grampositives Bakterium ist.

6. Präparat nach Anspruch 5, dadurch **gekennzeichnet,** daß das Bakterium zur Gattung Streptococcus gehört.

7. Präparat nach Anspruch 6, dadurch **gekennzeichnet,** daß das Bakterium Streptococcus sanguis ist.

8. Präparat nach Anspruch 6, dadurch **gekennzeichnet,** daß das Bakterium Streptococcus salivarius ist.

9. Re- oder umkombiniertes DNA-Molekül bestehend aus Segmenten der DNA aus verschiedenen Genomen, die außerhalb von lebenden Zellen endweise miteinander verbunden sind und die die Fähigkeit besitzen, einen Wirt zu transformieren und in diesem gehalten zu werden, und deren Abkömmlingen, **gekennzeichnet** durch eine DNA-Sequenz, die gewählt wird aus:

(a) einem 3.0 kb EcoRi-Fragment der im Bacillus circulans BC-8 (FERM BP-733) vorhandenen DNA, und die α-1,3 Glukan-3-glukanhydrolase enkodiert;
(b) DNA-Sequenzen, die zu dem v. a. DNA-Fragment hybridisieren und auf die Expression für α-1,3 Glukan-3-glukanhydrolyse kodieren, und
(c) DNA-Sequenzen, die auf die Expression für ein α-1,3 Glukan-3-glukanhydrolase-Enzym kodieren von der Art, die hierfür auf die Expression durch eine beliebige der v. a. DNA-Sequenzen und -Fragmente kodiert,
wobei die DNA-Sequenzen und -Fragmente wirksam an eine Expressionkontrollsequenz im rekombinierten DNA-Molekül gebunden sind.

10. Rekombiniertes DNA-Molekül nach Anspruch 9, dadurch **gekennzeichnet,** daß das Molekül einen Klonierungsträger aufweist, der zumindest eine Restriktionsendonuklease-Erkennungsstelle besitzt, wobei die DNA-Sequenz an einer der Erkennungsstellen oder zwischen zwei derartige Stellen eingefügt wird.

11. Rekombiniertes DNA-Molekül nach Anspruch 10, dadurch **gekennzeichnet,** daß die Expressionkontrollsequenz ebenfalls in den Klonierungsträger eingefügt wird.

12. Einzelliger Wirt, der mit zumindest einem rekombinierten DNA-Molekül nach einem der vorhergehenden Ansprüche 9 bis 11 transformiert ist.

13. Transformierter Wirt nach Anspruch 12, dadurch **gekennzeichnet,** daß der Wirt E. coli ist.

14. Transformierter Wirt nach Anspruch 12, dadurch **gekennzeichnet,** daß der Wirt ein grampositives Bakterium ist.

15. Transformierter Wirt nach Anspruch 14, dadurch **gekennzeichnet,** daß das Bakterium zur Gattung Streptococcus gehört.

16. Transformierter Wirt nach Anspruch 15, dadurch **gekennzeichnet,** daß das Bakterium Streptococcus sanguis ist.

17. Transformierter Wirt nach Anspruch 15, dadurch **gekennzeichnet,** daß das Bakterium Streptococcus salivarius ist.

18. Re- oder umkombiniertes DNA-Molekül bestehend aus Segmenten der DNA aus verschiedenen Genomen, die außerhalb von lebenden Zellen endweise miteinander verbunden sind und die die Fähigkeit besitzen, einen Wirt zu transformieren und in diesem gehalten zu werden, und deren Abkömmlingen, **gekennzeichnet** durch eine DNA-Sequenz, die gewählt wird aus:

(a) einem Fragment der DNA, die im Bacillus circulans BC-8 (FERM BP-733) oder in der Bakteriumgattung vorhanden ist, die mit CB-8 bezeichnet und unter FERM BP-995 eingetragen ist und die α-1,6 Glukan-6-glukanhydrolase kodiert,
(b) DNA-Sequenzen, die zu dem v. a. DNA-Fragment hybridisieren und auf die Expression für α-1,6 Glukan-6-glukanhydrolase kodieren, und
(c) DNA-Sequenzen, die auf die Expression für ein α-1,6 Glukan-6-glukanhydrolase-Enzym kodieren von der Art, die hierfür auf die Expression durch eine beliebige der v. a. DNA-Sequenzen und -Fragmente kodiert,
wobei die DNA-Sequenzen und -Fragmente wirksam an eine Expressionkontrollsequenz im rekombinierten DNA-Molekül gebunden sind.

EP 0 195 672 B1

## FIG. 1

$$----\blacktriangleright 3)-\alpha-D-Glcp-(1\!\!\left[\!\!\blacktriangleright 3)-\alpha-D-Glcp-(1\right]_x\!\!\blacktriangleright 3)-\alpha-D-Glcp-(1\!\!\left[\!\!\blacktriangleright 3)-\alpha-D-Glcp-(1\right]_y\!\!\blacktriangleright----$$

$$\underset{6}{\uparrow}\quad\quad\quad\quad\quad\quad\quad\quad\quad\quad\quad\underset{6}{\uparrow}$$

$$\left[\alpha-D-Glcp\right]_m\underset{6}{\overset{1}{\uparrow}}\quad\quad\quad\quad\left[\alpha-D-Glcp\right]_n\underset{6}{\overset{1}{\uparrow}}$$

$$\alpha-D-Glcp\underset{}{\overset{1}{\uparrow}}\quad\quad\quad\quad\quad\quad\alpha-D-Glcp\underset{}{\overset{1}{\uparrow}}$$

$$1\,(x+y=7;\ m+n=3;\ m=0\sim3)$$

## FIG. 2

pYEJOOI

Lac O
Lac
Synthetic Promoter
Hind III
EcoRI
Hind III
Am$^r$
Cm$^r$
Tet$^r$

## FIG. 3

pGB 301
9.8kb

Kpn I
Hpa I
Hind III
Pvu II
Ava II
Hind III
Ava II
Cop
Rep
Em$^r$
Hind III
Hpa II
BspRI
Cm$^r$
Bcl I
BslE II

9, 8, 7, 6, 5, 3, 2, 1

# FIG. 4

## FIG. 5

## FIG. 6

(a)

(b)